# EUROPEAN PATENT APPLICATION

(11) **EP 4 166 189 A1**
(43) Date of publication of application: **19.04.2023**
(21) Application number: 21202810.4
(22) Date of filing: 15.10.2021
(51) Int. Cl.: A61N 1/36

(54) **ACTIVE CONTROL OF INTRACOCHLEAR STIMULATION**

(71) Applicant: Oticon Medical A/S, 2765 Smørum (DK)
(72) Inventor: VAIARELLO, Yannik, 06220 Vallauris (FR); STAHL, Pierre, 06220 Vallauris (FR); GNASIA, Dan, 06220 Vallauris (FR)
(74) Representative: Demant

(57) **Abstract**

A cochlear implant is disclosed. The cochlear implant is configured to provide electrical stimulation to auditory nerve fibers of a cochlea of a recipient of the cochlear implant. The cochlear implant may comprise an interface configured to provide audio stimulation information based on an external signal, an electrode lead including a plurality of electrodes configured to provide electrical stimulation the auditory nerve fibers based on the audio stimulation information, a differential power supplier configured to provide an anodic current and a cathodic current based on the audio stimulation information, and a mode unit connected to one or more electrodes of the plurality of electrodes, and wherein the mode unit may be configured to set the one or more electrodes into a mode of a plurality of modes based on the audio stimulation information, and wherein the plurality of modes includes an active mode, and in the active mode the one or more electrodes receives the anodic current or the cathodic current.

## Description

### FIELD

The present disclosure relates to a cochlear hearing aid system. More particularly, the disclosure relates to provide a stimulation scheme with an improved stimulation rate by actively controlling an amplitude of both a positive charged pulse and a negative charged pulse that is transmitted to one or more electrodes of an electrode lead of the cochlear hearing aid system.

### BACKGROUND

A cochlear hearing aid implant (CI) is a device that contains electrodes inserted in an inner ear (the cochlea) of a patient to recover the sensation of audition to people suffering from severe to profound hearing loss. CIs are bypassing most of the functional hearing chain and generate series of electrical pulses inside the cochlea to initiate action potentials from the hair cells. Depending on their implementation, they can either be totally implanted or composed of two main parts. A first part is the sound processor, often placed near the ear. It contains microphones that capture the environmental sound, which is processed in real time into a series of codes usable by a second part, implanted into the patient. The implant receives both power and sound information through radiofrequency from the sound processor, and generates electrical pulses sent into the cochlea via electrodes inside the cochlea.

One of many issues of the cochlear hearing aid implant relates to the performance of the electrodes, and to be more specific, when an electrode is activated, the electrode generates a wide electrical field that spreads within the cochlea. This results in broad and unfocused excitations of the neurons and leads to decrease in hearing perceptions of the recipient. Different techniques were found to focus the electrical field of an active electrode, for example, by controlling the electrode electrical states (i.e. stimulation modes), changing the electrode shape or material (e.g. black platinum electrode or polymer electrodes). The most efficient and commonly used stimulation mode is monopolar, which is, one intracochlear electrode being activated, one extracochlear reference electrode being grounded, and all other electrodes put in a high impedance state. When the activated intracochlear electrode is activated a current returning path is generated between the activated electrode and the grounded extracochlear reference electrode. In the monopolar mode the stimulation pulses are actively controlled both in the anodic phase of the stimulation pulse and in the cathodic phase of the stimulation pulse, and that results in a more controlled returning path between the activated electrode and the grounded extracochlear electrode. In a common ground mode one intracochlear electrode is activated and the remaining intracochlear electrodes are grounded. In the common ground mode the current returning path is more complex as when one intracochlear electrode is activated a current returning path is generated between the activated electrode and each of the grounded intracochlear electrodes. That provides uncontrolled current returning paths in both phases of the stimulation pulses. To provide a more controlled returning paths during the cathodic phase a capacitor is provided to each of the intracochlear electrodes for the purpose of obtaining a capacitively discharge of the stimulation pulses. The advantage of the common ground mode is that the stimulation field of the activated intracochlear electrode is more focused in view of the monopolar mode, and thereby, the excitations of the neurons is more focused leading to an improved hearing perception of the recipient. But to overcome the disadvantages of the complexity in the returning paths the design of the implant becomes more complex.

Therefore, there is a need to provide a solution that addresses the above-mentioned problems. In particular, there is a need to provide a solution that allows controlled returning paths and focused stimulation fields without complex current returning paths.

### SUMMARY

An aspect of the present disclosure is to obtain a more controllable charge phase (i.e. an anodic phase) and discharge phase (cathodic phase) of a stimulation pulse provided by a cochlear hearing aid system.

A further aspect of the present disclosure is to obtain a more controllable charge phase and discharge phase while also improving a stimulation rate of a cochlear hearing aid system.

An even further aspect of the present disclosure is to obtain a more focused stimulation field of an activated intracochlear electrode while the other previously mentioned aspects are also obtained.

To obtain the following aspects, a cochlear implant for providing electrical stimulation to auditory nerve fibers of a cochlea of a recipient of the cochlear implant is disclosed. The cochlear implant may comprise an interface configured to provide audio stimulation information based on an external signal, an electrode lead including a plurality of electrodes configured to provide electrical stimulation the auditory nerve fibers based on the audio stimulation information, a differential power supplier configured to provide an anodic current and a cathodic current based on the audio stimulation information, and a mode unit connected to one or more electrodes of the plurality of electrodes, and wherein the mode unit may be configured to set the one or more electrodes into a mode of a plurality of modes based on the audio stimulation information, and wherein the plurality of modes includes an active mode, and in the active mode the one or more electrodes receives the anodic current or the cathodic current.

The cochlear implant receives the audio stimulation information via the interface, and the audio stimulation information may include information about electrical stimulation pulses which the cochlear implant is providing to the recipient via the plurality of electrodes. The information may include temporal fine structure information, such as an amplitude level, pulse width, a phase level and/or a frequency representing a part of an acoustical wave received by a microphone of the cochlear hearing aid system. Via the interface the cochlear implant may receive multiple audio stimulation information representing, where each of the multiple audio stimulation information represent an electrical stimulation pulse. The multiple audio stimulation information may be forwarded to the plurality of electrodes according to a stimulation scheme. The stimulation scheme provides the electrical stimulation pulses to the plurality of electrodes in a sequential manner meaning that no electrodes of the plurality of electrodes can stimulate at the same time. When an electrode is in the active mode, i.e. the electrode is providing an electrical stimulation based on the received electrical stimulation pulse in an anodic phase, and after providing the electrical stimulation the same electrode needs to be discharged in a cathodic phase to ensure charge recovery. In known solutions the electrode is being discharged capacitively, and during the discharging the electrode is not able to enter another active mode. Unfortunately, that leads to a reduced stimulation rate. In the present disclosure, the problem is solved by the differential power supplier which provides a controlled charge and discharge phase by providing respectively the anodic current and the cathodic current to the one or more electrodes of the plurality of electrodes during stimulation of auditory nerve fibers and during discharging of the cochlea, respectively. The mode unit may be configured to connect the one or more electrodes to the differential power supplier, and thereby, the mode unit is configured to select an electrode other than the electrode that has just been in the active mode during the anodic phase to be in a discharge phase during the cathodic phase. In this example, the selected electrode receives the cathodic current during the cathodic phase forwarded by the mode unit, and the level of the cathodic current determines the level of the discharge provided by the selected electrode. The determination of whether an electrode should be in the active mode is solely determined by the audio stimulation information and not on whether the electrode is discharging, and thereby, an improved stimulation rate is provided in comparison to known solutions. The differential power supplier is configured to control the level of charging and discharging of the electrode and that is an advantage over known art.

In one example, the cochlear implant may include a mode unit and a differential power supplier for each electrode of the plurality of electrodes. In another example, the cochlear implant may include a differential power supplier and a mode unit for a group of electrodes of the plurality of electrodes. In this example, the mode unit is configured to set multiple electrodes into different modes in a sequential manner and based on a stimulation scheme that is generated by the processing unit. The stimulation scheme informs about the electrical stimulation, such as stimulation levels of one or more electrodes, which electrodes to be set into the active mode, and the timing of each electrical stimulation levels. This information is provided by the audio stimulation information. By assigning multiple electrodes to the same differential power supplier and the mode unit results in a more compact solution than if the mode unit is configured to set only one electrode into a mode.

The plurality of modes includes the active mode, a high impedance mode, and a ground mode. In the active mode the one or more electrode receives an anodic current from the differential power supplier via the mode unit. In the ground mode the one or more electrode is connected to ground, and in the high impedance mode, the one or more electrode is connected to an impedance that is higher in the active mode and the ground mode.

The electrical stimulation includes a pulse current which is applied to the auditory nerve fibres and part of the current is returned back to an electrode of the plurality of electrodes that is connected to ground and/or back to one or more extracochlea ground electrode arranged outside the cochlea and/or to one or more intracochlear ground electrode. By setting an electrode to a high impedance mode would affect a return current to not be received by the electrode but instead by an electrode being connected to ground. It is possible to control the returning path of the return current, for example if an electrode which has a poor neural interface to the auditory nerve fibers it would be beneficial to connect that electrode to a high impedance. Furthermore, the controlling of the returning path implies may include controlling the level of returning current to an extracochlear ground electrode and/or to the intracochlear electrode for the purpose of controlling the level of stimulation mode, such as the level of monopolar or the level common ground mode. For example, by connecting the intracochlear electrodes and/or the one or more electrodes to high impedance mode and the extracochlear ground electrode to ground, then the stimulation mode becomes more monopolar, and the level of the monopolar stimulation mode would increase along with an increased number of intracochlear electrodes and/or with an increase number of electrodes being connected to a high impedance. The selection of stimulation mode and the level of the stimulation mode may be determined based on the audio stimulation information. Alternatively, the selection of stimulation mode may be determined based on a neural degeneration on a part of the cochlea of the recipient.

The plurality of modes may include a ground mode, and the mode unit may be configured to set the one or more electrodes to the ground mode by connecting the one or more electrodes to ground via a first ground mode unit based on the audio stimulation information. The first ground mode unit may be configured to connect the one or more electrodes to the ground either directly or indirectly via a first resistor based on the audio stimulation information. The first ground mode may include a digital switch circuitry, a ground and a first resistor, and wherein the digital switch circuitry may be configured to connect the one or more electrodes to the ground either directly or via a first resistor based on the audio stimulation information.

The cochlear implant may include a second ground mode unit configured to set one or more returning electrodes of the electrode into a ground mode by connecting the one or more returning electrodes to ground either directly or indirectly via a second resistor based on the audio stimulation information. The one or more returning electrodes may include one or more extracochlear ground electrode arranged outside the cochlea and/or one or more intracochlear ground electrode.

In general, the return current will go on the electrodes that shows less resistivity than others. This resistivity can be controlled by the first resistor and the second resistor such that the return current may be received by one or more extracochlear ground electrodes connected to ground, intracochlear electrode connected to ground and/or one or more electrodes of the plurality of electrodes connected to ground directly or via a resistor having a lower resistivity than the first resistor and/or the second resistore. By applying a resistor to an electrode the resistivity increases and the return current will tend to avoid this electrode, as the return current prefers lower resistive.

By applying the second resistor to an extracochlear ground electrode will allow the return current to favor more intracochlear paths for the purpose of obtaining a more focused electrical stimulation of the auditory nerve fibers. By applying the second resistor to an intracochlear will favor the extracochlear ground electrode path providing a more power efficient stimulation. By applying the second resistor to an intracochlear electrode and/or the first resistor to one or more electrodes of the plurality of electrodes can be used for steering the electrical stimulation towards different regions of the cochlea, for example for more apical or basal excitations. If resistors are coupled to basal located electrodes, the centroid of excitation will be shifted to the apex. If resistors are coupled to apical electrodes, the centroid of excitation will be shifted to the basal region. More extracochlear ground electrodes results in a more spread of the return current and which results in more auditory nerve fibers being stimulated by the same electrode being in the active mode. The recipient will perceive this as being louder than if less of the return current is spread, i.e., less extracochlear ground electrodes. That means, the anodic current level can be reduced, and which results in a reduced power consumption. However, the disadvantage of the spread is that the sound quality perceived by the recipient is reduced because of the unwanted stimulation of auditory nerve fibers. In a situation where the battery status is low it will be of an advantage that a processing unit of the cochlear hearing aid system is configured to control how many of the extracochlear ground electrodes should be set to ground mode rather than a high impedance mode depending on the battery status. By increasing the number of extracochlear ground electrodes being in the ground mode the battery lifetime is increased because less anodic current is needed to obtain the wanted loudness of the recipient.

If a recipient requires too much current to be fitted, this generally means that the recipient is suffering from poor neural interface. To overcome this issue a broader stimulation is wanted and is accomplished by connecting the one or more intracochlear electrodes and/or the one or more electrodes of the plurality of electrodes to ground via the second resistor and/or the first resistor, respectively. Some sound environment requires an accurate stimulation, for example, music. Music is a complex sound scene that contains a lot of information that need to be conveyed with precision. In that case, the extracochlear ground electrode must be connected to ground via the second resistor. A processor of the cochlear implant or an external unit connected inductively to the cochlear implant may be configured to automatically detect the complexity of an audio signal which the audio stimulation information is based.

The first resistor and/or the second resistor may be a variable resistor in which the resistivity is adjustable. The level of the resistivity may be determined based on the neural interface between the electrodes of the electrode lead and the neural auditory nerve fibers.

The one or more extracochlear ground electrodes, intracochlear electrodes and/or one or more electrodes of the plurality of electrodes may be connected to ground, i.e. being in a ground mode, for the purpose of receiving the return current. In a situation where the one or more extracochlear ground electrodes is connected ground and none of the plurality of electrodes a more power efficient stimulation is obtained as less power is need to obtain a comfortable electrical stimulation. In another situation where one or more electrodes of the plurality of electrodes and/or the one or more intracochlear electrodes are connected to ground a more focused electrical stimulation is obtained which means that unwanted stimulation of auditory nerve fibers will be reduced. This will inevitably result in an improved perception of the patient.

The cochlear implant may comprise a phase state switch configured to receive from the differential power supplier the anodic current and/or the cathodic current and forward the anodic current or the cathodic current based on a trigger signal to the mode unit. The trigger signal may be generated by a clock unit or the processing unit of the cochlear implant or cochlear implant system. The trigger signal may be a digital signalThe processing unit may trigger the phase state switch to forward the anodic current based on the audio stimulation information which informs about the required level of electrical stimulation. The differential power supplier may include an anodic current source configured to provide the anodic current and a cathodic current source configured to provide the cathodic current, and wherein the positive and the cathodic current source are connected to the phase state switch. The phase state switch provides a simple solution on how to forward either the positive or cathodic current to the mode unit.

The audio stimulation information may include temporal fine structures of the audio signal provided by a microphone and/or a streaming interface connected to the cochlear implant. The microphone and/or the streaming interface may be part of the cochlear implant or an external unit connected to the cochlear implant via an inductive interface. The interface that is configured to receive an external signal including audio stimulation may be an antenna, an inductive interface, or a microphone. The external signal may be an inductive signal or an acoustical signal. The audio stimulation information includes information about one or more electrical stimulation signals to the one or more electrodes. The information about the one or more electrical stimulation signals includes power level and/or, amplitude and/or phase and/or timing and/or frequency.

The second ground mode may be configured to set the one or more returning electrodes of the electrode lead to a high impedance mode, and where the high impedance mode includes an impedance which is higher than in the ground mode.

The differential power supplier may be configured to generate a current in a cathodic phase which is opposed in phase to a current generated in an anodic phase. The current is then forwarded to the mode unit and then to an electrode of the plurality of electrodes. In the anodic phase, an electrode of the plurality of electrodes is in the active mode configured to receive the current while the one or more intracochlear electrodes, the one or more extracochlear ground electrodes and/or one or more of the plurality of electrodes are connected to ground. In the cathodic phase, the electrode which in the anodic phase received the current may then receive another current with an opposite phase in relation to the current which the electrode received in the previous phase, and the one or more intracochlear electrodes, the one or more extracochlear ground electrodes and/or the one or more electrodes of the plurality of electrodes are still connected to ground. In another example when being in the cathodic phase, the electrode which in the anodic phase received the current may once again be in the active mode configured to receive another current which is in-phase with the previous current, and another electrode of the plurality of electrodes is configured to receive a current having an opposite phase to the current of the electrode which received the current in the anodic phase while the one or more intracochlear electrodes, the one or more extracochlear ground electrodes and/or the one or more electrodes of the plurality of electrodes are still connected to ground. When applying an electrical stimulation pulse in the anodic phase it is important to add the cathodic phase for ensuring balance charges in the cochlea.

A cochlear implant system may be disclosed including the cochlear implant and optionally an external unit, wherein the external unit and/or the cochlear implant may include one or more microphones, a signal processing unit, and a battery. The external unit may be arranged on an opposite side of the skin as the cochlear implant.

The signal processing unit may be configured to receive the audio stimulation information and to determine whether the connection to ground should be via the first resistor and/or the second resistor and/or to determine the resistor levels of the first resistor and/or the second resistor by determining whether a power level of an electrical stimulation to be applied to the one or more electrodes is above a maximum power level threshold.

The signal processing unit may be configured to receive the audio stimulation information and to determine whether the connection to ground should be via the second resistor and/or to determine the resistor level of the second resistor by determining whether the audio stimulation information relates to music.

The one or more electrodes may be in the active mode, one or more other electrodes of the plurality of electrodes may be in ground mode and directly connected to ground, and the one or more extracochlear ground electrode may be connected to ground via the second resistor which results in that the one or more other electrodes receives more return current from the stimulation provided by the one or more electrodes than the one or more extracochlear ground electrode. This results in a more focused electrical stimulation which is an advantage when the audio stimulation information relates to music.

The one or more electrodes may be in the active mode, one or more other electrodes of the plurality of electrodes or the one or more intracochlear ground electrodes may be in ground mode and connected to ground via the first resistor or the second resistor, respectively, and the one or more extracochlear ground electrode may be directly connected to ground provides that the one or more extracochlear ground electrode receives more return current from the stimulation provided by the one or more electrodes that is in the active mode than the one or more other electrodes being grounded.

The one or more electrodes may be in an active mode configured to receive the positive or the cathodic current within a first time period, and within the first time period the one or more extracochlear ground electrodes and/or the one or more intracochlear ground electrodes is grounded. Furthermore, within the first time period one or more other electrodes of the plurality of electrodes are in high impedance mode or grounded either directly or indirectly via the first resistor. The one or more electrodes in active mode may be grounded in a second time period, and within the second time period the one or more extracochlear ground electrodes and/or the one or more intracochlear ground electrodes and/or an electrode of the plurality of electrodes not been in the active mode during the first time period may be in active mode configured to receive a current opposite in phase of the current applied to the one or more electrode being in the active mode during the first time period, and wherein the first time period is before the second time period. In this example, the stimulation rate of the applied electrical stimulation pulses is improved as the charging or discharging in the second time period is provided via an electrode which is not going to be in the active mode during the second time period.

For improving the control of the current spread around an electrode being in active mode a group of grounded electrodes is arranged around the active electrode. A first group of electrodes of the plurality of electrodes may be in ground mode and assigned to a first extracochlear ground electrode of the one or more extracochlear ground electrodes, and wherein a second group of electrodes of the plurality of electrodes may be in ground mode assigned to a second extracochlear ground electrode of the one or more extracochlear ground electrodes. An electrode of the plurality of electrodes may be in the active mode and surrounded by at least two electrodes of one of the first group and the second group, the return current from the electrode in active mode is received partially by the at least two grounded electrodes and the assigned extracochlear ground electrode. In this example, the current spread around the active electrode is finer controlled. For improving the controlling of the current spread the first ground mode unit and the second ground mode unit are configured to shift between connection to ground via the first resistor and the second resistor, respectfully, and direct connection to ground.

The plurality of electrodes may be arranged along the electrode lead, and when the electrode lead is implanted in the cochlea of the recipient, the electrode lead has a basal end and an apical end. The plurality of electrodes may be arranged between the basal end and the apical end. In one example, an electrode of the plurality of electrodes is activated, i.e., being in the active mode, and is arranged at one of basal end or apical end asymmetrical return current is provided by the electrical stimulation. To remove asymmetrical return current at least one intracochlear ground electrodes is arranged at a most basal end and/or a most apical end of the electrode lead and is grounded. When an electrode at one of basal end or apical end is activated, the grounded electrode of the plurality of electrodes that is arranged next to the active electrode and the intracochlear ground electrode at one of basal end or apical end is grounded a symmetrical return current is provided via the intracochlear ground electrode and the grounded electrode of the plurality of electrodes.

### BRIEF DESCRIPTION OF DRAWINGS

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1A illustrates an example of a cochlear hearing aid system;
FIG. 1B illustrates an example of a cochlea with an electrode lead:
FIGS. 2A to 2D illustrate examples of a cochlear implant;
FIGS 3A to3C illustrate another examples of a cochlear implant;
FIGS 4A and 4B illustrate another examples of a cochlear implant;
FIGS 5A and 5B illustrate examples of an electrode lead; and
FIG 6 illustrates an example of a stimulation scheme.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in a In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant.

A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly running an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

A "cochlear implant system" represents a particular type of a "hearing system" comprising an external unit, which receives acoustic sound and processes the acoustic sound into a coded audio, and an implantable unit which receives the coded audio signal.

A Cochlear hearing aid implant typically includes i) an external part for picking up and processing sound from the environment, and for determining sequences of pulses for stimulation of the electrodes in dependence on the current input sound, ii) a (typically wireless, e.g. inductive) communication link for simultaneously transmitting information about the stimulation sequences and for transferring energy to iii) an implanted part allowing the stimulation to be generated and applied to a number of electrodes, which are implantable in different locations of the cochlea allowing a stimulation of different frequencies of the audible range. Such systems are for example described in US 4,207,441 and in US 4,532,930.

In an aspect, the hearing device comprises multi-electrode array e.g., in the form of a carrier comprising a multitude of electrodes adapted for being located in the cochlea in proximity of an auditory nerve of the user. The carrier is preferably made of a flexible material to allow proper positioning of the electrodes in the cochlea such that the electrodes may be inserted in cochlea of a recipient. Preferably, the individual electrodes are spatially distributed along the length of the carrier to provide a corresponding spatial distribution along the cochlear nerve in cochlea when the carrier is inserted in cochlea.

In still a further aspect, the functions may be stored on or encoded as one or more instructions or code on a tangible computer-readable medium. The computer readable medium includes computer storage media adapted to store a computer program comprising program codes, which when run on a processing system causes the data processing system to perform at least some (such as a majority or all) of the steps of the method described above, in the and in the claims.

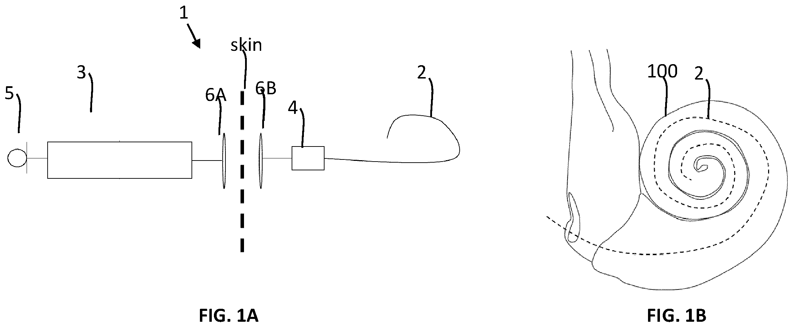

FIG. 1A illustrates an example of a cochlear hearing aid system 1 which includes an external unit 3 and a cochlear implant 4 both arranged on an opposite side of the skin of the recipient of the cochlear hearing aid system 1. In this example, the external unit 3 includes a microphone 5 and an inductive interface 6A configured to communicate inductively with an inductive interface 6B of the cochlear implant 4. In this example, the inductive interface is configured to provide an audio stimulation information based on an external signal. In another example, the microphone 5 may be arranged in the cochlear implant 4, and in this example, the interface which provides the audio stimulation information may be the microphone and/or the inductive interface 6B. In both examples, the cochlear implant includes an electrode lead 2 that includes a plurality of electrodes configured to provide electrical stimulation of auditory nerve fibers of cochlea.

FIG. 1B illustrates an example of a cochlea 100 with the electrode lead 2. The end of the electrode lead 2 which is arranged closest to an apical end is denoted as being an apical end, and the end of the electrode lead 2 which is arranged closest to a basal end of the cochlea 100 is denoted as a basal end of the electrode lead 2.

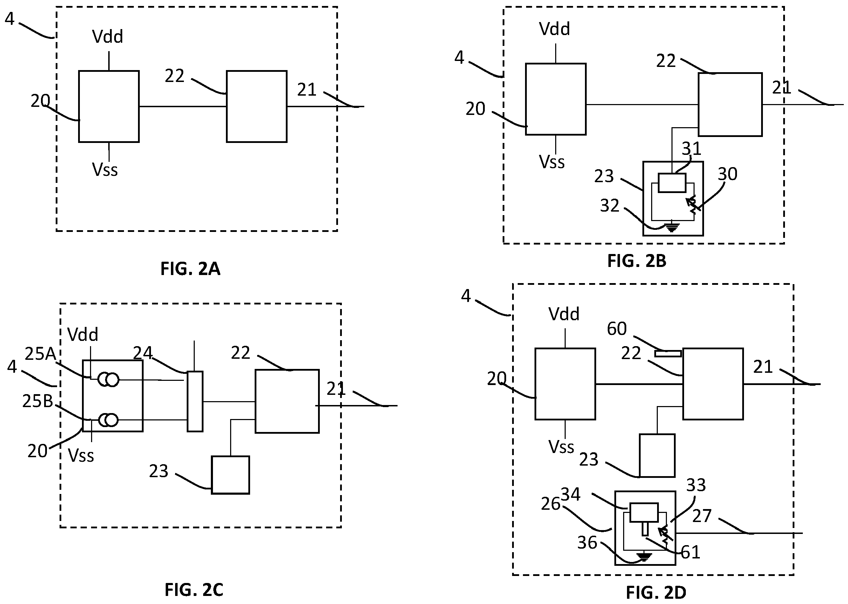

FIGS. 2A, 2B, 2C and 2D illustrate different examples of the cochlear implant 4 that includes an interface (5, 6B) (not shown) configured to provide audio stimulation information based on an external signal which may be acoustical or an inductive signal. The cochlear implant 4 includes a differential power supplier 20 configured to provide an anodic current and a cathodic current based on the audio stimulation information. A mode unit 22 is configured to receive the current and to be connected to one or more electrodes 21 of the plurality of electrodes 30. The mode unit 22 is configured to set the one or more electrodes 21 into a mode of a plurality of modes based on the audio stimulation information, and the plurality of modes may include an active mode, and in the active mode the one or more electrodes receives the anodic current or the cathodic current via the mode unit. In FIG. 2B, the cochlear implant 4 includes a first ground mode unit 23 that is connected to the mode unit 22. The mode unit 22 and the first ground mode unit 23 are configured to connect the electrode 21 to a ground 32 directly or via a first resistor 30. In this specific example, the first resistor has a variable resistivity level, i.e. the first resistor 30 is a variable resistor. In another example, the first resistor 30 may have a fix resistivity level. The shifting between a direct connection to ground 32 and a connection to ground 32 via the first resistor 30 is provided by a digital switch circuitry 31. The shifting is performed by the digital switch circuitry 31 and controlled by a processing unit which may be part of the cochlear implant 4 or the external unit 3. In FIG. 2C the differential power supplier 20 includes an anodic current source 25A configured to generate the anodic current and a cathodic current source 25B configured to generate the cathodic current, and the anodic current or the cathodic current is received by a phase state switch 24. The phase state switch is configured to forward the anodic current and/or the cathodic current to the mode unit based on a trigger signal. The cochlear implant 4 or the external unit 3 may include a clock unit (not shown) configured to provide the trigger signal. The processing unit may trigger the phase state switch to forward the anodic current based on the audio stimulation information which informs about the required level of electrical stimulation signals forwarded to an electrode of the plurality of electrodes.

The audio stimulation information includes information about an electrical stimulation signal such as stimulation level in the form of amplitude level and/or phase level, a frequency or an electrode number, and the timing of the electrical stimulation in relation to other electrical stimulations defined by a stimulation scheme.

In FIG. 2D the cochlear implant 4 includes a second ground mode unit 26 that is configured to set one or more returning electrodes 27 of the electrode lead into a ground mode by connecting the one or more returning electrodes 27 to ground 36 either directly or indirectly via a second resistor 33 based on the audio stimulation information. In this specific example, the second resistor has a variable resistivity level, i.e. the second resistor 33 is a variable resistor. In another example, the second resistor 33 may have a fix resistivity level. The shifting between a direct connection to ground 36 and a connection to ground 36 via the second resistor 33 is provided by a digital switch circuitry 34. The shifting is performed by the digital switch circuitry 34 and controlled by a processing unit which may be part of the cochlear implant 4 or the external unit 3. The one or more returning electrodes 27 includes one or more extracochlear ground electrode arranged outside the cochlea and/or one or more intracochlear ground electrode arranged within the cochlea. Furthermore, the mode unit 22 is configured to set the electrode 21 into a high impedance mode 60, and the second ground mode unit 26 is configured to set the one or more return electrodes 27 into a high-impedance mode 61.

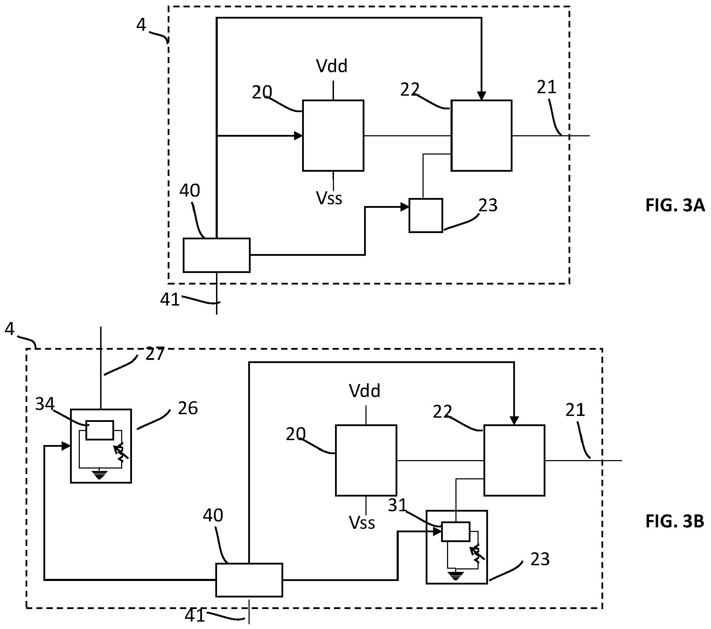

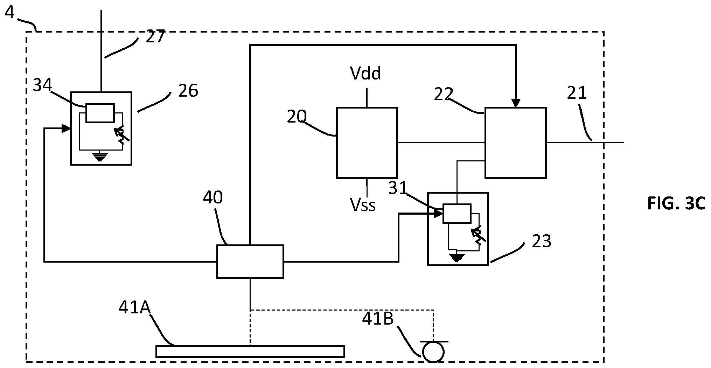

FIGS 3A to 3C illustrate an example of the cochlear implant 4 which includes a processing unit 40. In another example, the processing unit may be located in the external unit 3. In FIG. 3A, the processing unit 40 is configured to control the differential power supplier 20 and/or the phase state switch unit 24 (not shown), the mode unit 22 and the first ground mode unit 23. The processing unit is connected to the interface configured to receive the audio stimulation information from the interface 41,and based on the audio stimulation information the processing unit 40 is configured to control whether the differential power supplier 20 shall provide an anodic current or a cathodic current to an electrode 21 of the plurality of electrodes. Furthermore, the processing unit 40 is configured to receive multiple audio stimulation information in a stimulation scheme and to control the differential power supplier 20, the phase state switch 24, the mode unit 22 and the first ground mode unit 23 based on the stimulation scheme. In FIG. 3B, the processing unit 40 is configured to control the digital switch circuitry (31,34) of the first 23 and second 26 ground mode unit. In FIG. 3C the interface (41A, 41B) includes an inductive interface 41A and/or a microphone 41B configured to provide the audio stimulation information either based on an inductive signal provided by an external unit 3 or based on an acoustical wave received by the microphone 41B. Not illustrated, the interface (41A, 41B) may include multiple microphones.

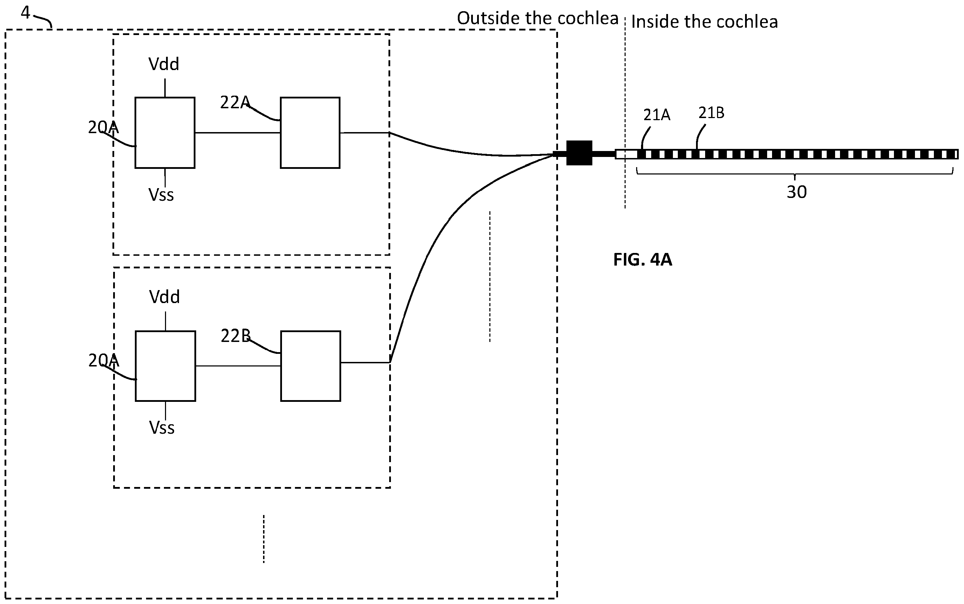

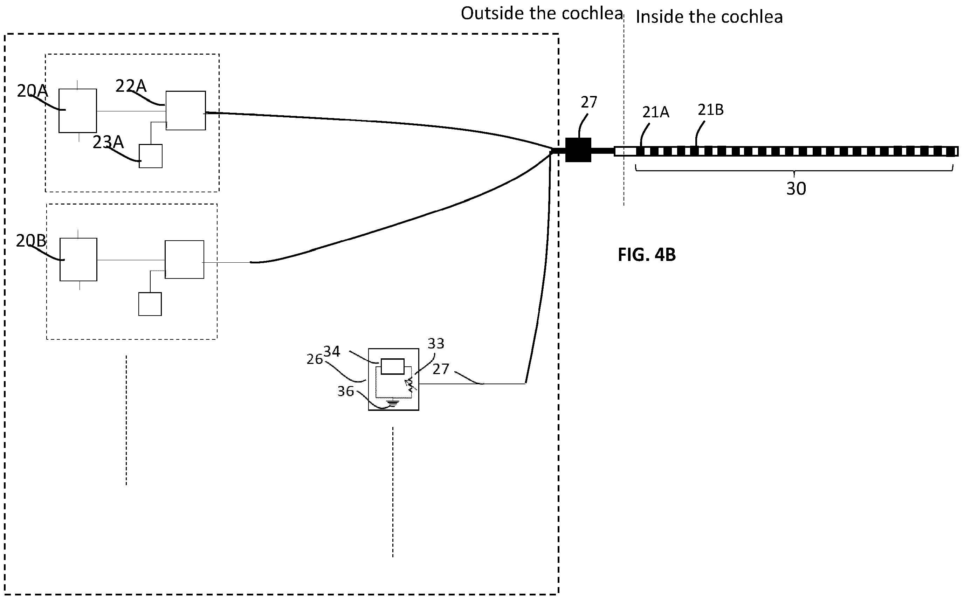

FIGS. 4A to 4C illustrate another example of the cochlear implant 4, an in the examples, each electrode (21A, 21B) of the plurality of electrodes 30 is connected to a mode unit (22A, 22B) and a differential power supplier (20A,20B). Each of the electrodes (20A,20B) may be set into an active mode by the mode unit (22A, 22B) in a sequential manner determined by the audio stimulation information and/or a stimulation scheme. In this example, the first electrode 21A may be set into an active mode by the first mode unit 22A as the audio stimulation information includes an electrical stimulation pulse having a frequency which the first electrode 21A is assigned to Furthermore, the audio stimulation information includes another electrical stimulation pulse which has a different frequency that is assigned to the second electrode 21B, and the stimulation scheme determines that it is either the first (21A) or the second (21B) electrode which will first be set into the active mode followed by the other electrode (21A,21B). In FIG. 4B the electrode lead includes one return electrode 27 which in this example is an extracochlear ground electrode located outside the cochlea when the plurality of electrodes 30 is inserted into the cochlea. Furthermore, the cochlear implant 4 includes a second ground mode unit 26 which in this example is connected to the extracochlear ground electrode 27. In one example, within a period of time the first electrode 21A is in active mode receiving an anodic current, and the second electrode 21B is connected to ground directly and not via a first resistor 30 (not shown), and the extracochlear ground electrode is connected to ground via a second resistor 33. In this example, the return current provided by the electrical stimulation which is provided by the first electrode 21A is mainly received by the second electrode 21B resulting in a more focused electrical stimulation. Furthermore, for a subsequent time period, the first electrode 21A is kept in the active mode receiving a cathodic current, and the second electrode 21B and the return electrode 27 are in the same mode as in the previous time period. In the subsequent time period balanced charges is obtained within the cochlea. In another example, the stimulation scheme includes audio stimulation information for two stimulation pulses which both are assigned to the first electrode 21A because of the frequency content of the two stimulation pulses. Within a given time period the first electrode 21A is set to the active mode and receiving an anodic current having a current level determined by the audio stimulation information that relates to the first stimulation pulse, and in a subsequent time period, the first electrode 21B is still in the active mode and receiving another anodic current having a current level determined by the audio stimulation information relating to the second stimulation pulse. During the subsequent time period the second electrode 21B is set to the active mode receiving a cathodic current where the cathodic current level is determined by the audio stimulation information relating to the first stimulation pulse. In this example, the return electrode 27 is set to ground either via the second resistor 33 or directly.

The electrode lead 2 may include more than one return electrode 27. For example, the electrode lead 2 may include one or more extracochlear ground electrode and/or one or more intracochlear ground electrode.

### 50 intracochlear ground electrode

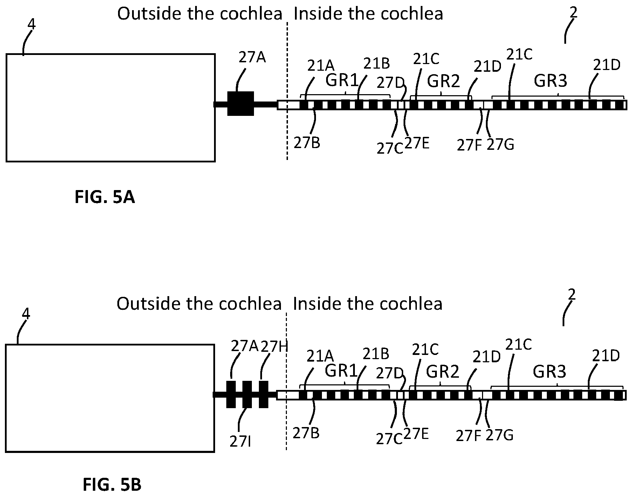

FIGS 5A and 5B illustrate different examples of the electrode lead 2. In Fig. 5A the electrode lead 2 includes an extracochlear ground electrode, and three groups (GR1, GR2, GR3) of electrodes (21A, 21B, 21C, 21D) and where each of the groups are separated by two or more intracochlear ground electrodes (27V, 27E, 27F, 27G). Between each electrodes of a group an intracochlear ground electrode 27B is arranged. By having an intracochlear ground electrode between each of the electrodes provides the possibility of focusing the stimulation provided by the electrode being in an active mode. In FIG. 5B, the extracochlear ground electrode (27A, 27H, 271) are divided into three separate extracochlear ground electrode, and where each of the three extracochlear ground electrode is assigned to one of the three groups of electrodes (GR1, GR2, GR3). In this example, when an electrode of the first group GR1 is in active mode, the extracochlear ground electrode assigned to the electrode of the first group is set into a ground mode by the second ground unit 26, wherein the other extracochlear ground electrode may be assigned to a high impedance mode. By having more than one extracochlear ground electrode improves the safety of the design of the electrode lead if one of the extracochlear ground electrode breaks down. In this example, all electrodes of the plurality of electrodes are assigned to at least one of the multiple extracochlear ground electrodes.

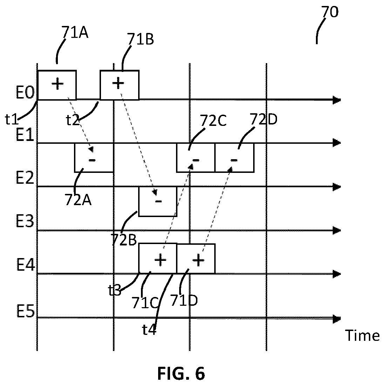

FIG. 6 illustrates an example of a stimulation scheme 70. The stimulation scheme includes audio stimulation information of the plurality of electrodes 70. In this example the stimulation scheme includes audio stimulation information of anodic current (71A-71D) and cathodic current (72A-72D). In this example, electrode E0 is set into an active mode at a first time receiving an anodic current 71A, and at a subsequently time to the first time t1 electrode E2 is set to active mode receiving a cathodic current 72A. The level of the cathodic current is determined by the anodic current 71A or by an audio stimulation information not relating to the previous anodic current 71A . During the delivering of the cathodic current, electrode E0 is yet again set into an active mode receiving another anodic current 71B at a second time t2. Subsequently to the second time t2, electrode E2 is set into an active mode receiving a cathodic current. Similar is seen at time t3 and t4, where electrode E4 receives an anodic current at two consecutive times (t3,t4), and electrode E2 provides the cathodic current for creating balanced chargers in the cochlear.
As already outlined above, the above described method, including all corresponding exemplary embodiments, for a cochlear implant system may be implemented in software.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. A cochlear implant for providing electrical stimulation to auditory nerve fibers of a cochlea of a recipient of the cochlear implant, wherein the cochlear implant comprising:
• an interface configured to provide audio stimulation information based on an external signal,
• an electrode lead including a plurality of electrodes configured to provide electrical stimulation of the auditory nerve fibers based on the audio stimulation information,
• a differential power supplier configured to provide an anodic current and a cathodic current based on the audio stimulation information, and
• a mode unit connected to one or more electrodes of the plurality of electrodes, and wherein the mode unit is configured to set the one or more electrodes into a mode of a plurality of modes based on the audio stimulation information, and wherein the plurality of modes includes an active mode, and in the active mode the one or more electrodes receives the anodic current or the cathodic current.

2. A cochlear implant according to claim 1, comprising a phase state switch configured to receive from the differential power supplier the anodic current and/or the cathodic current and forward the anodic current or the cathodic current based on a trigger signal to the mode unit.

3. A cochlear implant according to claim 2, comprising a clock unit configured to provide the trigger signal to the phase state switch based on the audio stimulation information.

4. A cochlear implant according to claim 2, wherein the differential power supplier includes an anodic current source configured to provide the anodic current and a cathodic current source configured to provide the cathodic current, and wherein the positive and the cathodic current source are connected to the phase state switch.

5. A cochlear implant according to any of the previous claims, wherein the audio stimulation information includes information about one or more electrical stimulation signals, and wherein the information about the one or more electrical stimulation signals includes stimulation level and/or, amplitude and/or phase and/or timing and/or frequency.

6. A cochlear implant according to any of the previous claims, comprising a first ground mode unit connected to the mode unit, and the mode unit and the first ground mode unit are configured to set the one or more electrodes to a ground mode of the plurality of modes by connecting the one or more electrodes to ground based on the audio stimulation information.

7. A cochlear implant according to claim 6, wherein the first ground mode includes a digital switch circuitry, a ground and a first resistor, and wherein the digital switch circuitry is configured to connect the one or more electrodes to the ground either directly or via a first resistor based on the audio stimulation information.

8. A cochlear implant according to any of the claims, comprising a second ground mode unit configured to set one or more returning electrodes of the electrode lead into a ground mode by connecting the one or more returning electrodes to ground either directly or indirectly via a second resistor based on the audio stimulation information.

9. A cochlear implant according to claim 8, wherein the one or more returning electrodes includes one or more extracochlear ground electrode arranged outside the cochlea and/or one or more intracochlear ground electrode arranged within the cochlea.

10. A cochlear implant according to any of the previous claims, wherein the plurality of modes includes a high impedance mode, and the mode unit is configured to set the one or more electrodes to the high impedance mode by connecting the one or more electrodes to an impedance which is higher than in the active mode and the ground mode.

11. A cochlear implant according to claim 8, wherein the second ground mode is configured to set the one or more returning electrodes of the electrode lead to a high impedance mode, and where the high impedance mode includes an impedance which is higher than in the ground mode.

12. A cochlear implant according to any of claims 7 to 11, comprising a signal processing unit configured to receive the audio stimulation information and to determine whether the connection to ground should be via the first resistor and/or the second resistor and/or to determine the resistor levels of the first resistor and/or the second resistor by determining whether a power level of an electrical stimulation to be applied to the one or more electrodes being in the active mode is above a maximum power level threshold

13. A cochlear implant according to any of claims 8 to 12, comprising a signal processing unit configured to receive the audio stimulation information and to determine whether the connection to ground should be via the second resistor and/or to determine the resistor level of the second resistor by determining whether the audio stimulation information relates to music.

14. A cochlear implant according to claims 9, wherein the one or more electrodes is in the active mode, one or more other electrodes of the plurality of electrodes are in ground mode and directly connected to ground, and the one or more extracochlear ground electrode is connected to ground via the second resistor which provides that the one or more other electrodes receives more return current from the stimulation provided by the one or more electrodes than the one or more extracochlear ground electrode.

15. A cochlear implant according to claim 9, wherein the one or more electrodes is in the active mode, one or more other electrodes of the plurality of electrodes or the one or more intracochlear ground electrodes are in ground mode and connected to ground via the first resistor or the second resistor, respectively, and the one or more extracochlear ground electrode is directly connected to ground provides that the one or more extracochlear ground electrode receives more return current from the stimulation provided by the one or more electrodes that is in the active mode than the one or more other electrodes being grounded.

16. A cochlear implant according to claims 9 and 11, wherein the one or more electrodes in active mode receives the positive or the cathodic current within a first time period, and within the first time period the one or more extracochlear ground electrodes and/or the one or more intracochlear ground electrodes is grounded, and within the first time period one or more other electrodes of the plurality of electrodes are in high impedance mode or grounded either directly or indirectly, and the one or more electrodes in active mode is grounded in a second time period, and within the second time period the one or more extracochlear ground electrodes and/or the one or more intracochlear ground electrodes and/or an electrode of the plurality of electrodes not being in the active mode during the first time period is configured to receive a current opposite in phase of the current applied to the one or more electrode in the active mode in the first time period, and wherein the first time period is before the second time period.

17. A cochlear implant according to any claim 9, wherein a first group of electrodes of the plurality of electrodes being in ground mode is assigned to a first extracochlear ground electrode of the one or more extracochlear ground electrodes, and wherein a second group of electrodes of the plurality of electrodes being in ground mode is assigned to a second extracochlear ground electrode of the one or more extracochlear ground electrodes, and when at least an electrode of the plurality of electrodes is in active mode surrounded by at least two electrodes of one of the first group and the second group, the return current from the electrode in active mode is received partially by the at least two grounded electrodes and the assigned extracochlear ground electrode.
